# EUROPEAN PATENT SPECIFICATION

(11) **EP 2 854 828 B1**
(45) Date of publication and mention of the grant of the patent: **07.08.2019**
(21) Application number: 13794326.2
(22) Date of filing: 24.05.2013
(51) Int. Cl.: A61K 36/258, A61K 31/704, A61K 31/70

(54) **A METHOD OF PREPARING A PANAX SPP. PLANT EXTRACT WITH INCREASED CONTENT RATIO OF GINSENOSIDE RG3, RG5, AND RK1 PRODUCED BY MICROWAVE IRRADIATION**
VERFAHREN ZUR HERSTELLUNG VON PANAX SPP. PFLANZENEXTRAKT MIT ERHÖHTEM GEHALT VON GINSENOSID RG3, RG5 UND RK1, HERGESTELLT DURCH MIKROWELLENBESTRAHLUNG
PROCÉDÉ DE PRÉPARATION D' UN EXTRAIT DE GINSENG (PANAX SPP.) AYANT UNE TENEUR ACCRUE EN GINSÉNOSIDES RG3, RG5 ET RK1 PRODUITS PAR IRRADIATION PAR MICRO-ONDES

(30) Priority: 25.05.2012 KR 20120056238; 23.01.2013 KR 20130007651
(43) Date of publication of application: 08.04.2015
(73) Proprietor: Korea Institute of Science and Technology, Seoul 136-791 (KR)
(72) Inventor: HAM, Jungyeob, Gangneung-si Gangwon-do 210-100 (KR); KANG, Ki Sung, Daejeon 302-803 (KR); CHUNG, Bong Chul, Namyangju-si Gyeonggi-do 472-709 (KR); KWON, Hak Cheol, Seoul 135-240 (KR); PARK, Soon Hye, Gangneung-si Gangwon-do 210-758 (KR); CHOI, Pil Ju, Gangneung-si Gangwon-do 210-730 (KR); KIM, Su Nam, Gangneung-si Gangwon-do 210-756 (KR); YANG, Hyun Ok, Seoul 135-774 (KR)
(74) Representative: Gill, Siân Victoria
(86) International application number: PCT/KR2013/004562
(87) International publication number: WO 2013/176512

(56) References cited:
- KR-A- 20080 099 362
- KR-A- 20090 089 814
- KR-A- 20090 089 815
- KR-A- 20110 094 532
- KR-A- 20110 123 311
- US-A1- 2003 190 378
- US-A1- 2005 031 711
- US-A1- 2006 198 908
- YUPING BAI ET AL: "Microwave Degradation of Floatation-Enriched Ginsenoside Extract from Panax quinquefolium L. Leaf", JOURNAL OF AGRICULTURAL AND FOOD CHEMISTRY, vol. 57, no. 21, 11 November 2009 (2009-11-11), pages 10252-10260, XP055228749, US ISSN: 0021-8561, DOI: 10.1021/jf902153a
- WANG YU KIM ET AL: "Steaming of Ginseng at High Temperature Enhances Biological Activity", JOURNAL OF NATURAL PRODUCTS., vol. 63, no. 12, 1 December 2000 (2000-12-01), pages 1702-1704, XP055228891, US ISSN: 0163-3864, DOI: 10.1021/np990152b
- JUN YEON PARK ET AL: "Protective Effects of Processed Ginseng and Its Active Ginsenosides on Cisplatin-Induced Nephrotoxicity: In Vitro and in Vivo Studies", JOURNAL OF AGRICULTURAL AND FOOD CHEMISTRY, vol. 63, no. 25, 1 July 2015 (2015-07-01), pages 5964-5969, XP055228727, US ISSN: 0021-8561, DOI: 10.1021/acs.jafc.5b00782

## Description

### Technical Field

The present invention relates to a method of preparing a Panax spp. plant extract and more particularly, to a processed Panax spp. plant extract including 90% or more of Rg3, Rk1, and Rg5 with respect to a weight of ginsenoside Rb1, Rc, Rb2, Rd, Rg3, Rk1, and Rg5 by processing a Panax spp. plant extract.

### Background Art

Panax ginseng is a perennial plant belonging to the Panax species, Araliaceae family. Examples of Panax species plants having a similar efficacy to Panax ginseng include Panax quinquefolia, Panax notoginseng, Panax japonica, Panax Trifolia, Panax pseudoginseng, Panax vietnamensis, and the like. These Panax species plants contain dammarane-based saponin in common with 1 to 4 saccharide(s) combined to a dammarane backbone, unlike other plants. In particular, saponins contained at high concentration in ginseng, include ginsenosides Rb1, Rb2, Rc, Rd, Rg1, and Re. These saponins have a variety of pharmaceutical effects that greatly differ in types and intensities depending on the structures thereof. The dammarane-based saponin of the Panax species plants has protopanaxadiol (PPD) or protopanaxatriol (PPT) as a core nucleus, and may be classified as illustrated in Formula 1.

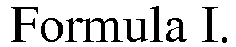

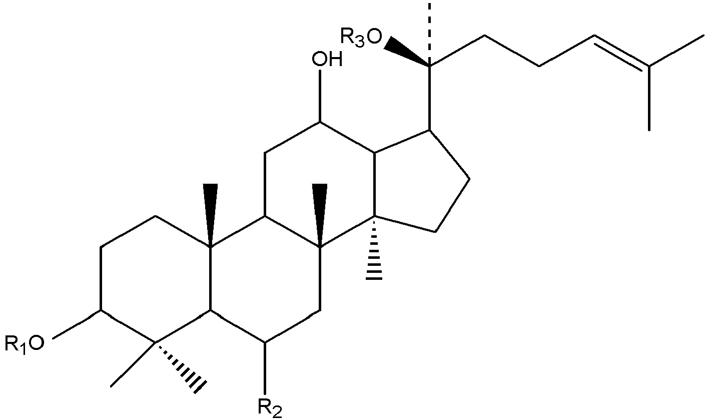

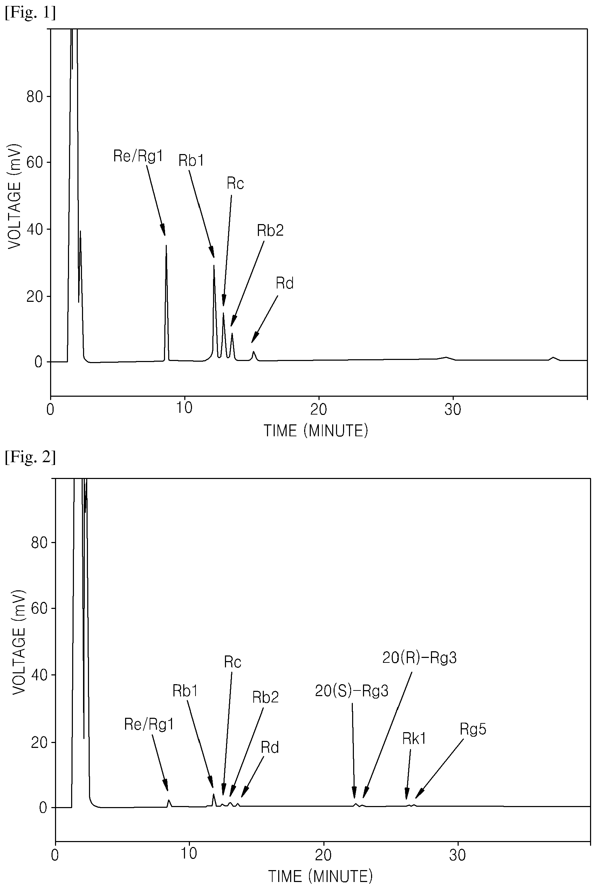

A dammarane-based saponin may be classified according to R1, R2, and R3 of FIG. 1 as below.

### Table 1

**[Table 1]**

| Group | ginsenoside | R₁ | R₂ | R₃ |
|---|---|---|---|---|
| Protopanaxadiol (PPD) | Ra1 | -Glc-Glc | -H | -Glc-Ara(pyr)-Xyl |
| | Ra2 | -Glc-Glc | -H | -Glc-Ara(fur)-Xyl |
| | Ra3 | -Glc-Glc | -H | -Glc-Glc-Xyl |
| | Rb1 | -Glc-Glc | -H | -Glc-Glc |
| | Rb2 | -Glc-Glc | -H | -Glc-Ara(pyr) |
| | Rb3 | -Glc-Glc | -H | -Glc-Xyl |
| | Rc | -Glc-Glc | -H | -Glc-Ara(fur) |
| | Rd | -Glc-Glc | -H | -Glc |
| | Rg3(20-R,S) | -Glc-Glc | -H | -H |
| | Rh2(20-R,S) | -Glc | -H | -H |
| | Rs1 | -Glc-Glc-Ac | -H | -Glc-Ara(pyr) |
| | Rs2 | -Glc-Glc-Ac | -H | -Glc-Ara(fur) |
| | Rs3 | -Glc-Glc-Ac | -H | -H |
| | malonyl-Rbl | -Glc-Glc-malonyl | -H | -Glc-Glc |
| | malonyl-Rc | -Glc-Glc-malonyl | -H | -Glc-Ara(fur) |
| | malonyl-Rd | -Glc-Glc-malonyl | -H | -Glc |
| | pseudoginsenoside F2 | -Glc | -H | -Glc |
| | notoginsenoside Fe | -Glc | -H | -Glc-Ara(fur) |
| Protopanaxatriol (PPT) | Re | -H | -OGlc-Rha | -Glc |
| | Ff | -H | -OGlc-Glc | -H |
| | Rg1 | -H | -OGlc | -Glc |
| | Rg2(20-R,S) | -H | -OGlc-Rha | -H |
| | Rh1(20-R,S) | -H | -OGlc | -H |
| | notoginsenoside R1 | -H | -OGlc-Xyl | -Glc |
| | notoginsenoside R2 | -H | -OGlc-Xyl | -H |
| | pseudoginsenoside F1 | -H | -OH | -Glc |
| | pseudoginsenoside F8 | -Glc-GlcAc | -OH | -Glc-Glc |
| | pseudoginsenoside F3 | -H | -OH | -Glc-Ara(pyr) |
| | Rh4 | -H | -OGlc | -methyl |

Rg5 and Rk1 are compounds that may be each respectively represented by Formulae 2 and 3, wherein R1 is -Glc-Glc, and R2 is -H.

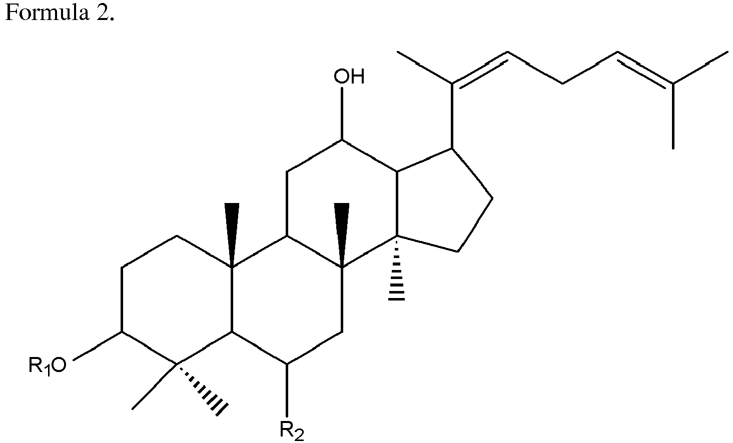

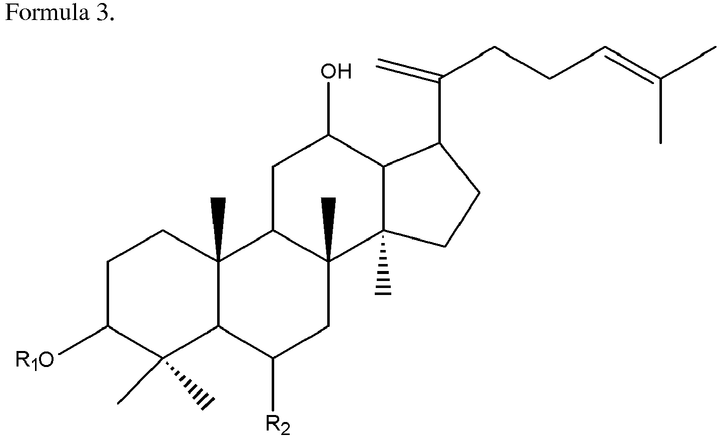

Ginsenoside Rg5 is known for its antioxidative effects, anticancer effects, a promotion of immune function, inhibitory effects on declining immune function and developing an anticancer drugs resistance as side-effects of anticancer drugs, a promotion of brain function and cognitive function such as memory and learning ability, prevention of dementia through the activation of brain cells, vasodilation, inhibitory effects on platelet aggregation, improving effects on dermatitisand/or psoriasis, and anti-inflammatory effects. Ginsenoside Rk1 is mainly known for its vasodilation effects, inhibitory effects on platelet aggregation, and improving effects on brain function and cognitive function such as memory and learning ability, prevention of dementia through the activation of brain cells, anti-aging and anti-cancer and cell regeneration. Ginsenoside Rg5 is known for its activities of inhibition of cancer cell metastasis, inhibition of platelet aggregation, anti-blood clot, inhibition of liver damage, anti-cancer drug, and inhibition of developing a anticancer drugs resistance.

Protopanaxadiol-type ginsenosides Rb1 and Rb2 may produce 20(S)-Rg3 and 20(R)-Rg3 when -Glc-Glc or -Glc-Ara(pyr) is deglycated at position 20. Also, Rg5 and Rk1 may be produced when dehydration occurs at position 20 of 20(S)-Rg3 and 20(R)-Rg3.

Studies on a ginseng extract having a sufficiently increased content ratio of ginsenoside Rg5 and Rk1 is needed. Also, since ginsenoside Rg5 and Rk1 may be derived from ginsenoside Rg3, a ratio of ginsenoside (Rg5 + Rk1)/(Rg3) is important in defining a content increase of Rg5 and Rk1. A ginseng extract having a sufficiently increased content ratio of ginsenoside Rg5, Rg3 and Rk1 based on the amount of protopanaxadiol type gensenoside, for example Rb1, Rc, Rb2, Rd, Rg3, Rk1, and Rg5 is needed.

US 2005/0031711 discloses a method of processing ginseng via heat treatment of a mixture of ingredients comprising ginseng and herbal drugs at a temperature of 70-120° C, which can improve the ratio of ginsenoside (Rg3+Rg5)/(Rb1+Rb2+Rc+Rd) to 10-45.

US 2006/0198908 discloses a ginseng preparation comprising high concentrations of ginsenosides (Rg3, Rg5, and Rh1). The preparation is prepared by adding vinegar of pH 2 to 4 to ginseng and heat-extracting the same for 0.5 to 24 hours.

Bai et al "Microwave Degradation of Floatation-Enriched Ginsenoside Extract from Panax quinquefolium L. Leaf" J. Agric. Food Chem. 2009, 57, 10252-10260 discloses a microwave irradiation degradation method for obtaining the ginsenosides Rg3, Rh2 and their aglycon 20(S)-protopanaxadiol. The method involves microwave irradiation degradation of the major ginsenosides from the Panax quinquefolium L. coupled with foam floatation.

KR 1020080099362 discloses a Panax species plant extract with a ratio of the ginsenoside (Rg3+Rg5+Rk1)/(Rb1+Rb2+Rc+Rd) over 1.0. The extract is prepared by heating at 70-150°C for 2-6 hours. The extract is dissolved in water, a C1-C4 low grade alcohol or a mixture thereof.

### Disclosure of Invention

### Technical Problem

The present invention provides a method of preparing the Panax spp. plant extract having an increased content ratio of ginsenoside Rg5 + Rk1 + Rg3.

The present disclosure also provides a pharmaceutical composition including the Panax spp. plant extract having an increased content ratio of ginsenoside Rg5 + Rk1 + Rg3.

The present disclosure also provides a health functional food composition including the Panax spp. plant extract having an increased content ratio of ginsenoside Rg5 + Rk1 + Rg3.

The present disclosure also provides a pharmaceutical composition including the Panax spp. plant extract for improving one of the side effects of anticancer drugs such as declining immune function and developing anticancer drugs resistance, improving antioxidation, anticancer, anti-inflammation, brain function or cognitive function, vasodilation, inhibition of platelet aggregation, improving dermatitis, or improving psoriasis.

According to an aspect of the present invention, there is provided a method of preparing a Panax spp. plant root extract containing 90% or more of Rg3, Rk1, and Rg5 with respect to a weight of ginsenoside Rb1, Rc, Rb2, Rd, Rg3, Rk1, and Rg5, wherein the method includes irradiating microwaves to a Panax spp. plant root or an extract thereof, wherein the irradiating of the microwaves is performed under pressure at a temperature in a range of 150°C to 190°C for 30 minutes to 90 minutes, wherein the irradiating of the microwaves is performed under a pressure in a range of 2 atm to 100 atm and the Panax spp. plant extract has a ratio of (Rg5 + Rk1)/(Rg3) that is at least 2.

### Solution to Problem

As used herein, the term "and/or" includes any and all combinations of one or more of the associated listed items. Expressions such as "at least one of," when preceding a list of elements, modify the entire list of elements and do not modify the individual elements of the list.

Hereinafter, one or more embodiments of the present invention will be described in detail with reference to the accompanying drawings.

Unless otherwise defined, all technical terms used herein have the same meaning as commonly understood by one of ordinary skill in the art to which the present invention pertains. In addition, exemplary methods or samples are described in the present specification, but it should be understood that those similar or equivalent thereto are also encompassed in the scope of the invention.

The inventors of the present invention studied a method of processing a Panax spp. plant extract, including ginseng, for significantly increasing a content of ginsenoside Rg3+Rg5+Rk1 in the Panax spp. plant extract compared to a conventional Panax spp. plant extract or a process product, and as a result, a method of increasing a content ratio of ginsenoside Rg3+Rg5+Rk1 by irradiating microwaves to a Panax spp. plant itself or a Panax spp. plant extract was developed. Particularly, a product obtained by reacting the Panax spp. plant extract in a microwave irradiator had a significantly increased content ratio of ginsenoside Rg3+Rg5+Rk1 compared to the case of the Panax spp. plant extract that is simply heat-treated. Also, the Panax spp. plant extract obtained by extracting with an extraction solvent after irradiating microwaves to a Panax spp. plant in a microwave irradiator had a significantly increased content ratio of ginsenoside Rg3+Rg5+Rk1 compared to the case of the Panax spp. plant extract that is simply heat-treated.

Therefore, according to an aspect of the present invention, provided is a method of preparing a Panax spp. plant root extract containing 90% or more of Rg3, Rk1, and Rg5 with respect to a weight of ginsenoside Rb1, Rc, Rb2, Rd, Rg3, Rk1, and Rg5, wherein the method comprises irradiating microwaves to a Panax spp. plant root or an extract thereof, wherein the irradiating of the microwaves is performed under pressure at a temperature in a range of 150°C to 190°C for 30 minutes to 90 minutes, wherein the irradiating of the microwaves is performed under a pressure in a range of 2 atm to 100 atm and the Panax spp. plant extract has a ratio of (Rg5 + Rk1)/(Rg3) that is at least 2.

An extract obtainable by irradiating microwaves to the Panax spp. plant extract or a Panax spp. plant extract obtainable by extracting with an extraction solvent after irradiating microwaves to a Panax spp. plant (hereinafter, referred to as "a microwave-irradiated process product") may contain 90% or more of Rg3, Rk1, and Rg5 with respect to a weight of ginsenoside protopanaxadiol, for example, Rb1, Rc, Rb2, Rd, Rg3, Rk1, and Rg5 obtainable by irradiating microwaves to the Panax spp. plant or the extraction thereof compared to the case when a Panax spp. plant extract is simply heat-treated (hereinafter, referred to as "a simple heat-treated process product". For example, a content of Rg3, Rk1, and Rg5 with respect to a weight of ginsenoside protopanaxadiol, for example, Rb1, Rc, Rb2, Rd, Rg3, Rk1, and Rg5 may be from 95% to 100%, from 98% to 100%, from 90% to 100%, from 99% to 100%, or 100%. Also, the microwave-irradiated process product may have a significantly high ratio of ginsenoside (Rg5 + Rk1)/(Rg3), and, for example, (Rg5+Rk1)/(Rg3) may be at least 2, at least 3, at least 4, or at least 5.

The microwave-irradiated process product has a higher content of ginsenoside Rg3+Rg5+Rk1, particularly Rg5 and Rk1 than the simple heat-treated process product and thus has an increased medicinal effect of ginsenoside Rg3+Rg5+Rk1, particularly of Rg5 and Rk1. Ginsenoside Rg3 is known for its anticancer effects, nerve protecting functions, inhibitory effects on platelet aggregation, antioxidant effects, anti-flammatory functions, kidney protecting functions, and antifatigue effects. Ginsenoside Rg5 is known for its antioxidative effects, anticancer effects, inhibitory effects on declining immune function and developing anticancer drug resistance as side-effects of anticancer drugs, improving effects on brain function and cognitive function, blood vessels expansion effects, inhibitory effects on platelet aggregation, improving effects on dermatitis and psoriasis, and antiinflammatory effects. Ginsenoside Rk1 is mainly known for its vasodilation effects inhibitory effects on platelet aggregation, and improving effects on brain function and cognitive function. Thus, the microwave-irradiated process product may have the effects listed above that are significantly increased compared to that of the simple heat-treated process product.

A pharmaceutical composition that includes the product of the claimed methods may be used for improving one of the side effects of anticancer drugs such as declining immune function and developing anticancer drugs resistance, improving antioxidation, anticancer, anti-inflammation, brain function or cognitive function, vasodilation inhibition of platelet aggregation, improving dermatitis, or improving psoriasis.

A health functional food composition may be prepared including the product of the claimed methods.

The health functional food as used herein refers to health functional foods that sufficiently have functionality and stability for the human body as newly defined by a law regarding health functional foods that was amended in 2008 and listed in regulations regarding the acceptance of functional raw materials of health functional foods prescribed in Korea Food & Drug Administration (KFDA) notification No: 2008-72.

The pharmaceutical composition and the health functional food composition according to the present disclosure are also collectively referred to herein as "compositions according to the present invention".

The microwave irradiation indicates thermal reaction heating a Panax spp. plant or a Panax spp. plant extract by irradiating microwaves. The microwave may be a radio wave having a wavelength in a range of about 1 m to about 1 mm, and a frequency in a range of about 300 MHz to about 300 GHz. The heating by using microwaves may vary depending on an amount of state of a reactant. The microwave irradiation is performed at a temperature in a range of 150°C to 190°C, for example, 160°C to 190°C, 170°C to 190°C, 180°C to 190°C, 150°C to 180°C, 150°C to 170°C, 150°C to 160°C, 170°C to 180°C, or 160°C to 170°C. Also, the microwave irradiation is performed for 30 minutes to 90 minutes, 30 minutes to 80 minutes, 30 minutes to 70 minutes, 30 minutes to 60 minutes, 30 minutes to 50 minutes, 30 minutes to 40 minutes, 40 minutes to 90 minutes, 50 minutes to 90 minutes, 60 minutes to 90 minutes, 70 minutes to 90 minutes, 80 minutes to 90 minutes, 50 minutes to 80 minutes, 60 minutes to 80 minutes, 70 minutes to 80 minutes, or 50 minutes to 70 minutes. The microwave irradiation is performed under a pressure in a range of 2 atm to 100 atm, 5 atm to 100 atm, or 10 atm to 100 atm. The microwave irradiation may be performed in a neutral solution, for example an aqueous solution. An output of the microwaves is not particularly limited and may be appropriately increased or decreased according to an amount of the reactant. For example, the output of the microwaves may be in a range of 50 W to 1000 W or 100 W to 700 W. Conditions for the microwave irradiation allow a content of Rg3, Rk1, and Rg5 to be 90% or more with respect to a weight of ginsenoside Rb1, Rc, Rb2, Rd, Rg3, Rk1, and Rg5 by irradiating microwave. Also, the conditions for the microwave irradiation allow a ratio of (Rg5 + Rk1)/(Rg3) to be at least 2, at least 3, at least 4, or at least 5. The resultant of the microwave irradiation may be used as it is, or may be used in the form of a material that is dried or freeze-dried. According to an embodiment of the present invention, the microwave irradiation may be performed on an aqueous solution including a Panax spp. plant or a Panax spp. plant extract for 30 minutes to 90 minutes at a temperature of 150°C to 190°C under a pressure of, for example, 2 atm to 100 atm, 5 atm to 100 atm, or 10 atm to 100 atm.

The Panax spp. plant may include at least one of ginsenoside protopanaxadiol, for example, Rb1, Rb2, Re, Rg1, Rc, and Rd. Examples of the Panax spp. plant may include, but are not limited to, Panax ginseng, Panax quinquefolia, Panax notoginseng, Panax japonica, Panax trifolia, Panax pseudoginseng, Panax vietnamensis, cultured roots thereof, heat-treated or enzyme-treated process products thereof, and combinations thereof. The heat-treated process products include red ginsengs or black ginsengs. For example, the Panax spp. plant may be ginseng.

The Panax spp. plant extract, which corresponds to the reactant of the microwave irradiation, may be an extract of an arbitrary Panax spp. plant including at least one of ginsenoside Rb1, Rb2, Rc, and Rd. The Panax spp. plant extract may further include Re, and/or Rg1. The extract of Panax spp. plant may be a crude extract or a product purified by additional solvent fraction or chromatography. For example, the extract of Panax spp. plant may be, for example, a crude extract of water, C₁-C₄ alcohol, or a mixture thereof of any Panax spp. plant; a solvent fraction of n-hexane, methylenechloride, ethylacetate, butanol or a mixture thereof of the crude extract; or a purified product of the solvent fraction.

The crude extract of water, C₁-C₄ alcohol, or a mixture thereof may be, for example, a crude extract of methanol or ethanol, and when extracted, the amount of solvent used may be about 5 to about 15 times greater than that of the Panax spp. plant, for example, about 10 times. After adding the solvent to the Panax spp. plant, the Panax spp. plant may be extracted using one of general methods such as heating extraction, ultrasonic extraction, and reflux extraction, particularly, ultrasonic extraction. In the extraction process, the temperature of the solvent may be about 50 to about 200 °C, about 50 to about 150 °C, about 80 to about 200 °C, or at about 120 °C, but it is not limited thereto. In addition, the extraction time may be about 2 to about 4 hours, about 2 to about 3 hours, about 3 to about 4 hours, or about 3 hours, but is not limited thereto. In addition, the extraction process may be performed once to five times, for example, three times, but it is not limited thereto. A crude extract obtained by the above-described method may be used as the extract of Panax spp. plant. Alternatively, a solvent fraction obtained by additionally extracting the crude extract with an organic solvent may be used as the extract of Panax spp. plant. The solvent fraction may be a fraction obtained by extracting the crude extract with methylenechloride, hexane, ethylacetate, butanol, or a mixture thereof, but it is not limited thereto.

A further purified product of the solvent fraction described above may be used as the extract of Panax spp. plant. For example, at least one of ginsenoside Rb1, Rb2, Re, Rg1, Rc, and Rd may be further purified by column chromatography.

The microwave-irradiated process product may contain 90% or more, 95% or more, or 98% or more of Rg3, Rk1, and Rg5 with respect to a total weight of ginsenoside in the obtained extract. The total weight of ginsenoside may be a total weight of protopanaxadiol, for example, Rb1, Rc, Rb2, Rd, Rg3, Rk1, and Rg5 or a total weight of Re, Rg1, Rb1, Rc, Rb2, Rd, Rg3, Rk1, and Rg5. The microwave irradiated process product is extracted by using a solvent and is not in a state of fraction.

As a result of irradiating microwaves to a Panax spp. plant extract or a Panax spp. plant, most of ginsenoside protopanaxadiol, for example, Rb1, Rc, Rb2, Rd, Rg3, Rk1, and Rg5 are converted to ginsenoside Rg3, Rk1, and Rg5, thus an increased content of ginsenoside Rg3, Rk1, and Rg5 in the Panax spp. plant extract or Panax spp. plant is confirmed. Also, a total weight of ginsenoside Rg3, Rk1, and Rg5 was significantly increased, and a content ratio of ginsenoside (Rg5 + Rk1)/(Rg3) was significantly increased compared to the case when the Panax spp. plant extract is simply heat-treated (see Example 1 and Table 2).

The pharmaceutical composition according to the present disclosure may be formulated in the form of a general pharmaceutical formulation known in the art. The pharmaceutical formulation may include an orally-administered formulation, an injection, a suppository, a transdermally-administered formulation, and transnasally-administered formulation. However, the pharmaceutical formulations are not limited to the above examples, and may be administered in the form of any formations.
Preferably, the pharmaceutical composition may be formulated in the form of formulations for oral administration, including liquid dosage forms, such as solutions, emulsions, suspensions, extracts, or syrups, and solid dosage forms, such as powders, granules, tablets, capsules, or pills.

When being formulated into each formulation, the pharmaceutical composition may be prepared by adding a pharmaceutically acceptable excipient or additive that is needed for the preparation of each formulation. For example, when formulating into a solid dosage form for oral administration, the pharmaceutically acceptable excipient may be at least one selected from a diluent, a lubricant, a binder, a disintegrant, a sweetener, a stabilizer, and a preservative. The excipient may be any excipients that are pharmaceutically acceptable. In particular, the excipient may be lactose, corn starch, soybean oil, microcrystalline cellulose, or mannitol, the lubricant may be magnesium stearate or talc, and the binder may be polyvinylpyrrolidone or hydroxypropylcellulose. In addition, the disintegrant may be calcium carboxymethyl cellulose, sodium starch glycolate, polacrilin potassium, or crospovidone. When formulating into a liquid dosage form for oral administration, various kinds of excipients, as well as a frequently used simple diluent, such as water or liquid paraffin, for example, a wetting agent, a sweetener, a flavoring agent, and a preservative, may be used. For example, the sweetener may be sucrose, fructose, sorbitol, or aspartame. The stabilizer may be sodium carboxymethyl cellulose, beta-cyclodextrin, bleached bees wax, or xanthan gum. The preservative may be methyl p-hydroxybenzoate, propyl p-hydroxybenzoate, or potassium sorbate. In addition, an additive of a solid or liquid oral formulation may be at least one selected from flavors, vitamins, and antioxidants. As a known flavoring agent in addition to the above-described ingredients, natural flavors such as Japanese apricot flavor, lemon flavor, pineapple flavor, and herb flavor; natural colors such as natural fruit juice, chlorophylin, or flavonoid; a sweetening ingredient such as fructose, honey, sugar alcohol, or sugar; or an acidifier such as citric acid or sodium citrate may be used in combination.

The general pharmaceutical formulations may be appropriately prepared by those of ordinary skill in the art by using a general method that is well known in the art.

The pharmaceutical composition according to an embodiment of the present disclosure may be administered several times such that a total daily dosage of the microwave-irradiated process product as an active ingredient is about 0.01 mg/kg to 10 g/kg, preferably, about 1 mg/kg to about 1 g/kg per adult. The dosage may be appropriately increased or decreased according to an administration route, the degree of disease progress, gender, age, body weight, or clinical diagnosis of experts. The pharmaceutical composition may be used alone or along with operation, hormone therapy, chemotherapy, and a biological response modifier therapy for improving an anticancer
drug side-effect selected from declining immune function and developing anticancer drugs resistance, antioxidation, anticancer, improving brain function or cognitive function, dilating blood vessels, inhibiting platelet aggregation, or improving dermatitis or psoriasis.

The health functional food composition may be formulated in the form of a general health functional food formulation known in the art.

The health functional food may be prepared in the form of general dosage forms such as powders, granules, tablets, pills, capsules, suspensions, emulsions, syrups, infusions, liquids and solutions, or extracts. In addition, the health functional food may be prepared in any forms of health functional foods such as meat, sausage, bread, chocolate, candies, snacks, confectionery, pizza, ramen, other noodles, gums, jelly, dairy products including ice cream, any kind of soups, beverages, tea, a health drink, alcoholic beverages, or a vitamin complex. To formulate the health functional food, a sitologically acceptable carrier or additive, and any carrier or additive known in the art that is used in the preparation of a formulation to be formulated may be used. Examples of the additive include a variety of nutritional supplements, a vitamin, an electrolyte, a flavoring agent, a coloring agent, pectic acid and salts thereof, alginic acid and salts thereof, organic acid, a protective colloidal thickener, a pH adjuster, a stabilizing agent, an antiseptic, glycerin, alcohol, and a carbonating agent used in a carbonated beverage. In addition, the additive may include a pulp for preparation of a natural fruit and vegetable juice. These additives may be used alone or in combination. A content of such an additive is inconsequential, but may be selected from a range of 0.01 parts to 0.1 parts by weight, based on 100 parts by weight of the composition of the invention.

In the health functional food composition, a content of the microwave-irradiated process product may be appropriately determined according to the usage purpose (prevention or improvement). In general, the content of the microwave-irradiated process product may be from 0.01 wt% to 15 wt% based on a total weight of the health functional food composition. When the health functional food composition is prepared as a beverage, the content of the microwave-irradiated process product may be 0.02 g to 10 g, preferably, 0.3 g to 1 g, based on 100 ml of the health functional food composition.

The beverage may further include other ingredients as well as the active ingredient, and may further include a variety of flavoring agents or natural carbohydrates that are generally used in beverages. Non-limiting examples of the natural carbohydrates include general carbohydrates such as monosaccharides (e.g., glucose and fructose), disaccharides (e.g., maltose and sucrose), polysaccharides (e.g., dextrin and cyclodextrin) and sugar alcohols such as xylitol, sorbitol, and erythritol. In addition, examples of the flavoring agent include natural flavoring agents (e.g., thaumatin and stevia extracts) and synthetic flavoring agents (e.g., saccharin and aspartame). The content of the natural carbohydrate may be generally about 1 g to about 20 g, preferably, about 5 g to about 12 g, based on 100 ml of the beverage.

### Advantageous Effects of Invention

Methods of the present invention, produce a processed Panax spp. plant extract that has a significantly high content ratio of ginsenoside Rg5+Rk1+Rg3 compared to a processed Panax spp. plant extract prepared by simple heat-treating. Thus, the processed Panax spp. plant extract prepared by the methods of the present invention may be used to manufacture a pharmaceutical composition and a health functional food composition with enhanced ginsenoside Rg5+Rk1+Rg3 effect, particularly Rg5 and Rk1 effect.

### Brief Description of Drawings

The above and other features and advantages of the present invention will become more apparent by describing in detail exemplary embodiments thereof with reference to the attached drawings in which:
FIG. 1 is a high-performance liquid chromatography (HPLC) chromatogram analyzing ginsenoside constitutions of a raw ginseng;
FIG. 2 is a HPLC chromatogram analyzing ginsenoside constitutions in a simple freeze-dried product of Cheong-Kwan-Jang's red ginseng extract pill plus™;
FIG. 3 is a HPLC chromatogram analyzing ginsenoside constitutions in a processed ginseng extract prepared by simply heat-treating a raw ginseng at a temperature of about 120°C for about 3 hours;
FIG. 4 is a HPLC chromatogram analyzing ginsenoside constitutions in a microwave-irradiated process product that is microwave process-treated at a temperature of 150°C at 100 W for 10 minutes;
FIG. 5 is a HPLC chromatogram analyzing ginsenoside constitutions in a microwave-irradiated process product that is microwave process-treated at a temperature of 150°C at 100 W for 20 minutes;
FIG. 6 is a HPLC chromatogram analyzing ginsenoside constitutions in a microwave-irradiated process product that is microwave process-treated at a temperature of 150°C at 100 W for 30 minutes;
FIG. 7 is a HPLC chromatogram analyzing ginsenoside constitutions in a microwave-irradiated process product that is microwave process-treated at a temperature of 150°C at 100 W for 40 minutes;
FIG. 8 is a HPLC chromatogram analyzing ginsenoside constitutions in a microwave-irradiated process product that is microwave process-treated at a temperature of 150°C at 100 W for 50 minutes;
FIG. 9 is a HPLC chromatogram analyzing ginsenoside constitutions in a microwave-irradiated process product that is microwave process-treated at a temperature of 150°C at 100 W for 60 minutes;
FIG. 10 is a HPLC chromatogram analyzing ginsenoside constitutions in a microwave-irradiated process product that is microwave process-treated at a temperature of 150°C at 100 W for 70 minutes;
FIG. 11 is a HPLC chromatogram analyzing ginsenoside constitutions in a microwave-irradiated process product that is microwave process-treated at a temperature of 150°C at 100 W for 80 minutes;
FIG. 12 is a HPLC chromatogram analyzing ginsenoside constitutions in a microwave-irradiated process product that is microwave process-treated at a temperature of 150°C at 100 W for 90 minutes;
FIG. 13 is a HPLC chromatogram analyzing ginsenoside constitutions in a microwave-irradiated process product that is extracted by using an extraction solvent after irradiating microwaves to a ginseng extract at a temperature of 130°C at 100 W for 30 minutes;
FIG. 14 is a HPLC chromatogram analyzing ginsenoside constitutions in a microwave-irradiated process product that is extracted by using an extraction solvent after irradiating microwaves to a ginseng extract at a temperature of 140°C at 100 W for 30 minutes;
FIG. 15 is a HPLC chromatogram analyzing ginsenoside constitutions in a microwave-irradiated process product that is extracted by using an extraction solvent after irradiating microwaves to a ginseng extract at a temperature of 160°C at 100 W for 30 minutes;
FIG. 16 is a HPLC chromatogram analyzing ginsenoside constitutions in a microwave-irradiated process product that is extracted by using an extraction solvent after irradiating microwaves to a ginseng extract at a temperature of 170°C at 100 W for 30 minutes;
FIG. 17 is a HPLC chromatogram analyzing ginsenoside constitutions in a microwave-irradiated process product that is extracted by using an extraction solvent after irradiating microwaves to a ginseng extract at a temperature of 180°C at 100 W for 30 minutes;
FIG. 18 is a HPLC chromatogram analyzing ginsenoside constitutions in a microwave-irradiated process product that is extracted by using an extraction solvent after irradiating microwaves to a ginseng extract at a temperature of 190°C at 100 W for 30 minutes; and
FIG. 19 is a HPLC chromatogram analyzing ginsenoside constitutions in a microwave-irradiated process product that is extracted by using an extraction solvent after irradiating microwaves to a ginseng extract at a temperature of 200°C at 100 W for 30 minutes.

### Best Mode for Carrying out the Invention

One or more embodiments of the present invention will now be described more fully with reference to the following examples. For the avoidance of doubt, it is confirmed that Examples 2, 3, 11, 12 and 17 do not fall within the scope of the claimed invention.

### Example 1: Preparation of ginseng extract

Panax ginseng roots used in the present invention were purchased from herbal medicine shops in the Geumsan ginseng market, Korea. 50% ethanol (1.5 L) was added to 200 g of finely cut ginseng root powder and the resultant mixture was reflux extracted at 70°C for 3 hours to obtain a 50% ethanol extract. Thereafter, the obtained 50% ethanol extract was dried under reduced pressure to vaporize the solvent therefrom to obtain 27 g of a dried extract including ginsenoside Rb1, Rb2, Rc, Rd, Rg1, and Re.

### Examples 2 to 10: Preparation of processed ginseng extract by using microwave (1)

The 50% ethanol extract prepared according to Example 1 was heat-treated. In particular, each of 200 mg of the ginseng dry extracts was added to 1 mL of water in a 10 mL container of a microwave irradiator(model no.: 908005) manufactured by CEM Company in USA. The ginseng dry extract was irradiated with microwaves in the sealed container at a temperature of 150°C and a power of 100 W (a frequency of 2455 MHz) for 10 minutes (Example 2 (comparative)), 20 minutes (Example 3(comparative)), 30 minutes (Example 4), 40 minutes (Example 5), 50 minutes (Example 6), 60 minutes (Example 7), 70 minutes (Example 8), 80 minutes (Example 9), and 90 minutes (Example 10). The microwave irradiated ginseng dry extracts were freeze-dried to obtain microwave-irradiated process products. A pressure for the microwave irradiation was 20 atm.

### Examples 11 to 17: Preparation of processed ginseng extract by using microwave (3)

Microwaves were irradiated to the 50% ethanol extract prepared according to Example 1 at several different temperatures.

In particular, each of 200 mg of the ginseng dry extracts obtained according to Example 1 was added to 1 mL of water in a 10 mL container of a microwave irradiator (model no.: 908005) manufactured by CEM in USA. The ginseng dry extract was irradiated with microwaves in the sealed container for 30 minutes at a power of 100 W (a frequency of 2455 MHz) and a temperature of 130°C (Example 11 (comparative)), 140°C (Example 12 (comparative)), 150°C (Example 4), 160°C (Example 13), 170°C (Example 14), 180°C (Example 15), 190°C (Example 16), or 200°C (Example 17 (comparative)). The microwave irradiated ginseng dry extracts were freeze-dried to obtain microwave-irradiated process products. A pressure for the microwave irradiation was 20 atm.

### Comparative Examples 1 and 2: Preparation of Cheong-Kwan-Jang's red ginseng extract pill plus™ freeze-dried product and simple heat-treated processed ginseng extract (2)

A Cheong-Kwan-Jang's red ginseng extract pill plus™ freeze-dried product and a simple heat-treated processed ginseng extract were prepared to compare compositions of the microwave-irradiated products prepared according to Examples 2 to 10 with conventional heat-treated processed ginsengs, as follows. A ginseng extract of Cheong-Kwan-Jang's red ginseng extract pill plus™ available in the market was freeze-dried to obtain a freeze-dried product of the Cheong-Kwan-Jang's red ginseng extract (Comparative Example 1). Next, a raw ginseng was processed, in other words, the 50% ethanol extract prepared according to Example 1 was heat-treated. 200 mg of the 50% ethanol extract was added to 1 mL of purified water and then simply heat-treated in an autoclave at a temperature of about 120°C for about 3 hours. The processed ginseng extract extracted in the same manner as in Example 1 was freeze-dried to obtain a simple heat-treated extract (Comparative Example 2).

### Experimental Example 1: Analysis of ginsenoside of processed ginseng extract

### (1) Experimental method

Saponins of the processed ginseng extracts prepared according to Examples 1 to 17 and Comparative Examples 1 and 2 were each analyzed by HPLC disclosed in the publication (Kwon et al., J. Chromatogr. A, 921(2), 335-339, 2001), by using as a control a standard solution containing each of ginsenosides Rb1, Rc, Rb2, Rd, 20(S)-Rg3, 20(R)-Rg3, Rg5, and Rk1. The saponin analysis was repeated three times to confirm reproducibility. An elution solvent A included acetonrile, water, and acetic acid at a ratio of 15:80:5 (vol/vol/vol), respectively. An elution solvent B included acetonrile and water at a ratio of 80:20 (vol/vol), respectively. The elusion solvents A and B were each filtered through a 0.45 µm membrane filter and were respectively used by pumping with two pumps. 10 µl of the standard solution was injected into a separation column, i.e., a reverse phase column (C18, 4.6 X 150 mm) by using a syringe, and an elution solvent containing 100 volume% of the elution solvent A and 0 volume% of the elution solvent B was made to flow through the reverse phase column at a flow rate of 1 ml/min. Then, volume% of the elution solvent A was gradually reduced to 70% (6minutes), 50% (18minutes), and 0% (30 minutes), and then, the content was maintained for 7 minutes. After the foregoing process, a peak of each constituent separated from the separation column was analyzed using an evaporative light scattering detector (ELSD).

### (2) Experimental results

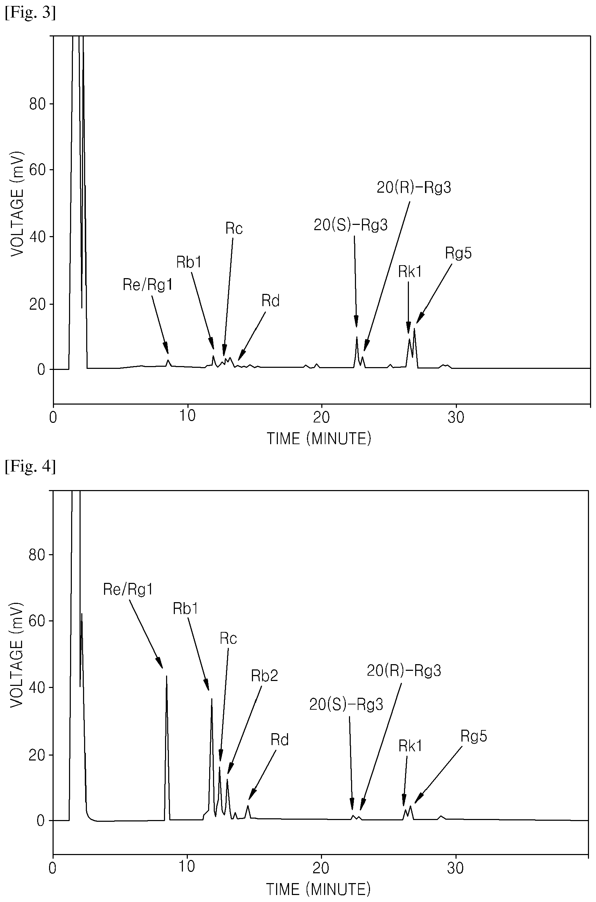

Peaks shown in FIG. 3 to 19 were obtained as the results of analyzing each constituent, which was separated from the separation column by the HPLC analysis of the processed ginseng extracts, using the ELSD.

FIG. 1 is a high-performance liquid chromatography (HPLC) chromatogram analyzing ginsenoside constitutions of a raw ginseng.

FIG. 2 is a HPLC chromatogram analyzing ginsenoside constitutions in a simple freeze-dried product of Cheong-Kwan-Jang's red ginseng extract pill plusTM.

FIG. 3 is a HPLC chromatogram analyzing ginsenoside constitutions in a processed ginseng extract prepared by simply heat-treating a raw ginseng at a temperature of about 120°C for about 3 hours.

FIG. 4 is a HPLC chromatogram analyzing ginsenoside constitutions in a microwave-irradiated process product that is microwave process-treated at a temperature of 150°C at 100 W for 10 minutes.

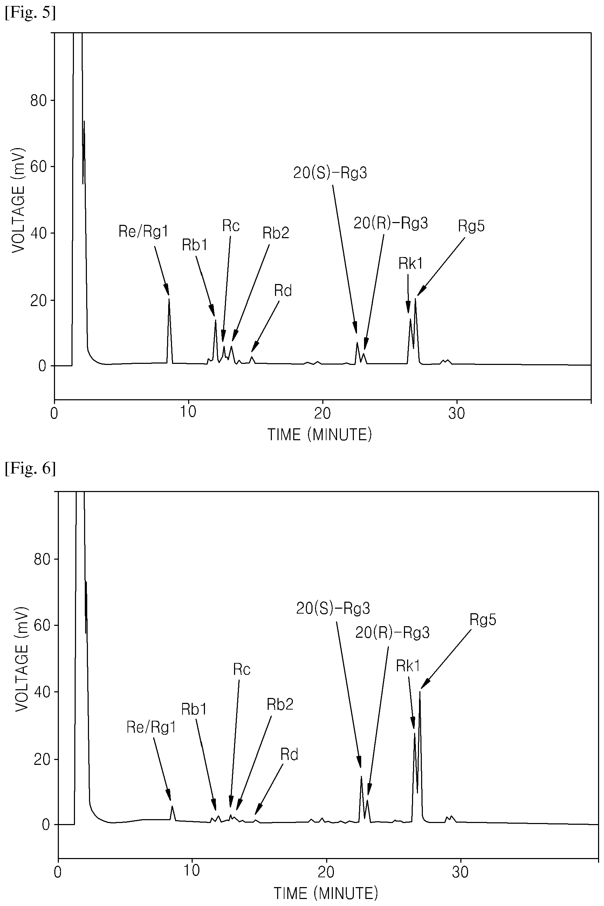

FIG. 5 is a HPLC chromatogram analyzing ginsenoside constitutions in a microwave-irradiated process product that is microwave process-treated at a temperature of 150°C at 100 W for 20 minutes.

FIG. 6 is a HPLC chromatogram analyzing ginsenoside constitutions in a microwave-irradiated process product that is microwave process-treated at a temperature of 150°C at 100 W for 30 minutes.

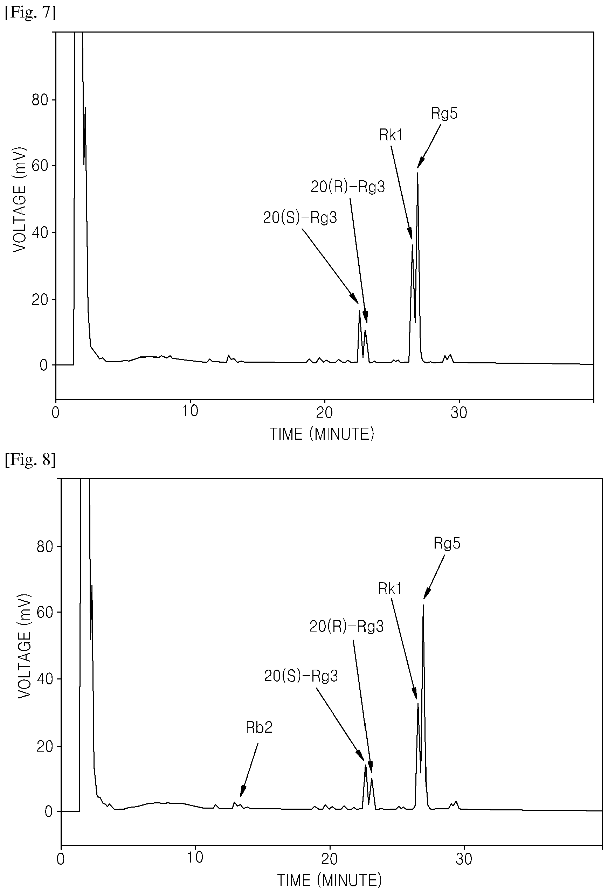

FIG. 7 is a HPLC chromatogram analyzing ginsenoside constitutions in a microwave-irradiated process product that is microwave process-treated at a temperature of 150°C at 100 W for 40 minutes.

FIG. 8 is a HPLC chromatogram analyzing ginsenoside constitutions in a microwave-irradiated process product that is microwave process-treated at a temperature of 150°C at 100 W for 50 minutes.

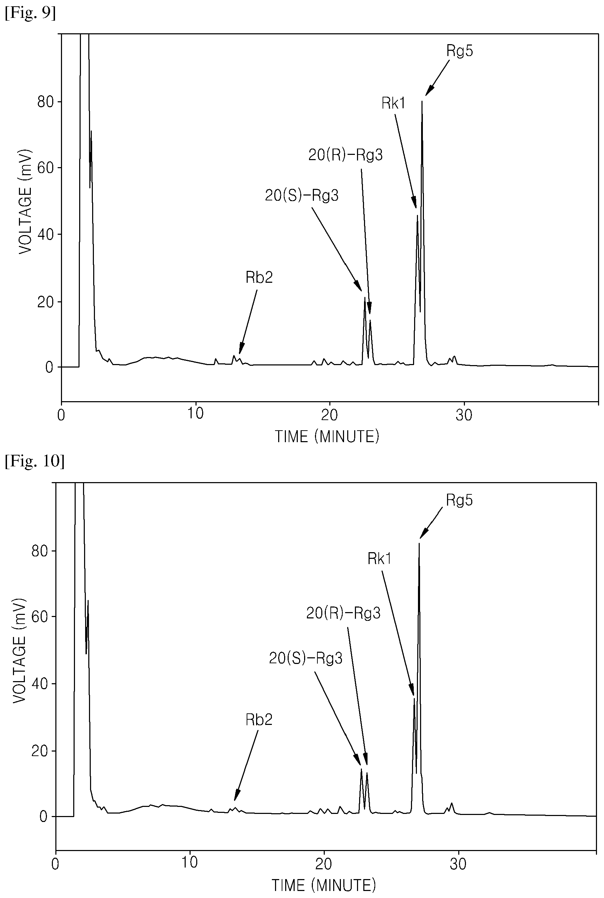

FIG. 9 is a HPLC chromatogram analyzing ginsenoside constitutions in a microwave-irradiated process product that is microwave process-treated at a temperature of 150°C at 100 W for 60 minutes.

FIG. 10 is a HPLC chromatogram analyzing ginsenoside constitutions in a microwave-irradiated process product that is microwave process-treated at a temperature of 150°C at 100 W for 70 minutes.

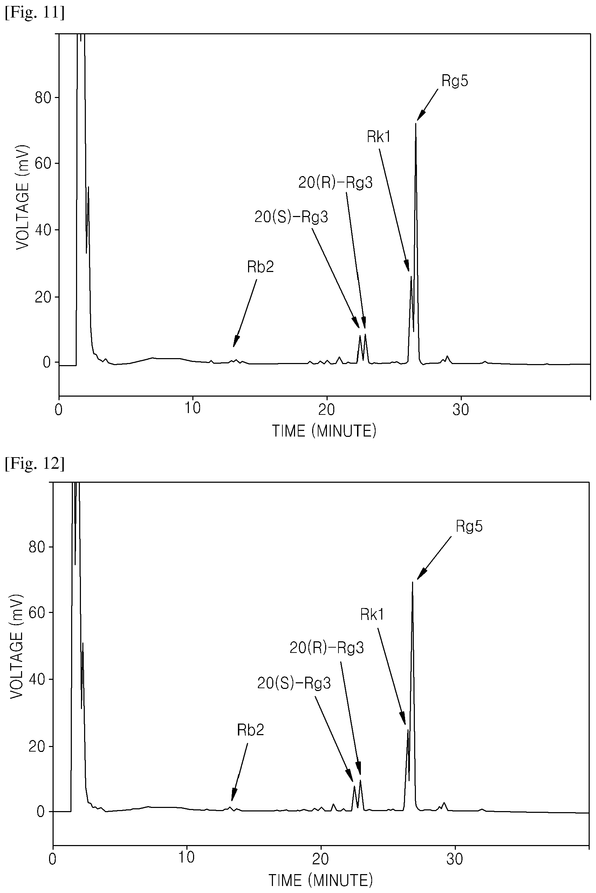

FIG. 11 is a HPLC chromatogram analyzing ginsenoside constitutions in a microwave-irradiated process product that is microwave process-treated at a temperature of 150°C at 100 W for 80 minutes.

FIG. 12 is a HPLC chromatogram analyzing ginsenoside constitutions in a microwave-irradiated process product that is microwave process-treated at a temperature of 150°C at 100 W for 90 minutes.

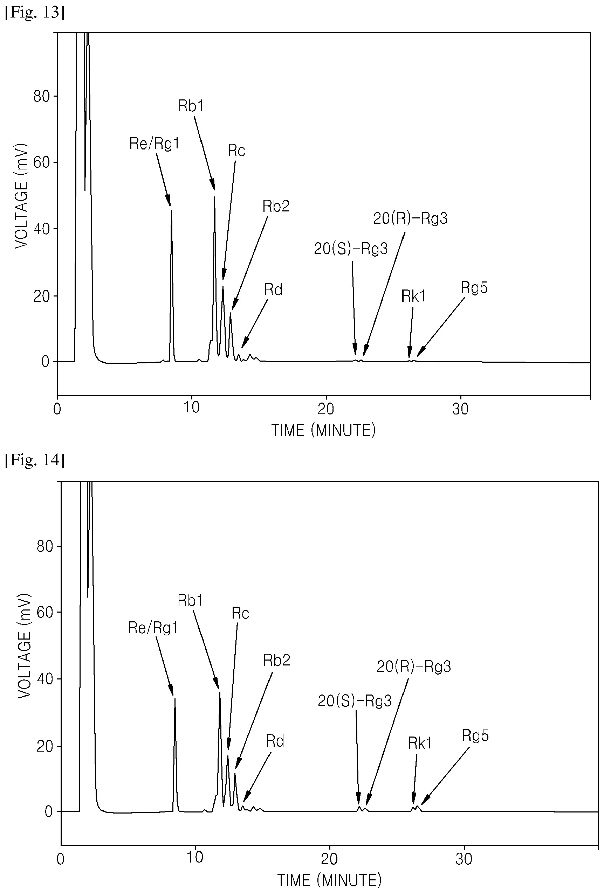

FIG. 13 is a HPLC chromatogram analyzing ginsenoside constitutions in a microwave-irradiated process product that is extracted by using an extraction solvent after irradiating microwaves to a ginseng extract at a temperature of 130°C at 100 W for 30 minutes.

FIG. 14 is a HPLC chromatogram analyzing ginsenoside constitutions in a microwave-irradiated process product that is extracted by using an extraction solvent after irradiating microwaves to a ginseng extract at a temperature of 140°C at 100 W for 30 minutes.

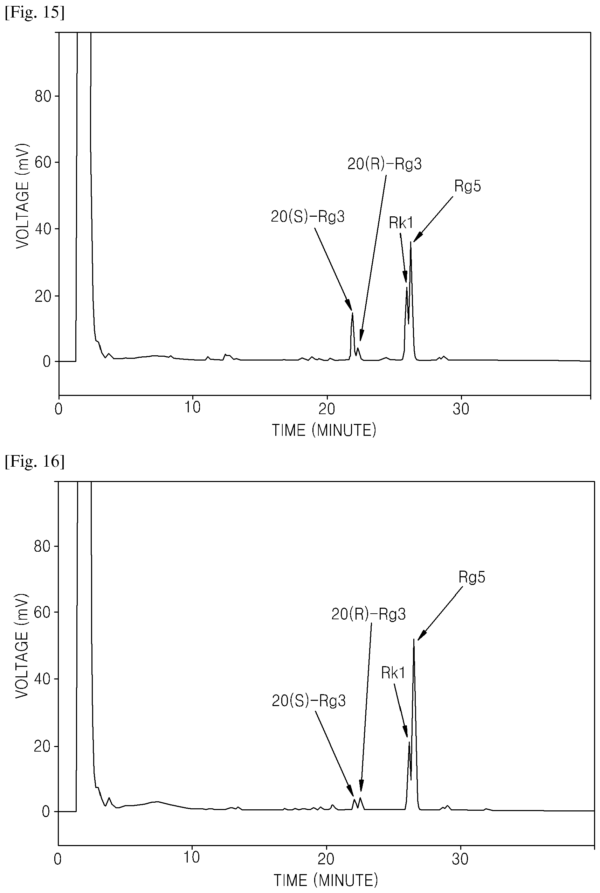

FIG. 15 is a HPLC chromatogram analyzing ginsenoside constitutions in a microwave-irradiated process product that is extracted by using an extraction solvent after irradiating microwaves to a ginseng extract at a temperature of 160°C at 100 W for 30 minutes.

FIG. 16 is a HPLC chromatogram analyzing ginsenoside constitutions in a microwave-irradiated process product that is extracted by using an extraction solvent after irradiating microwaves to a ginseng extract at a temperature of 170°C at 100 W for 30 minutes.

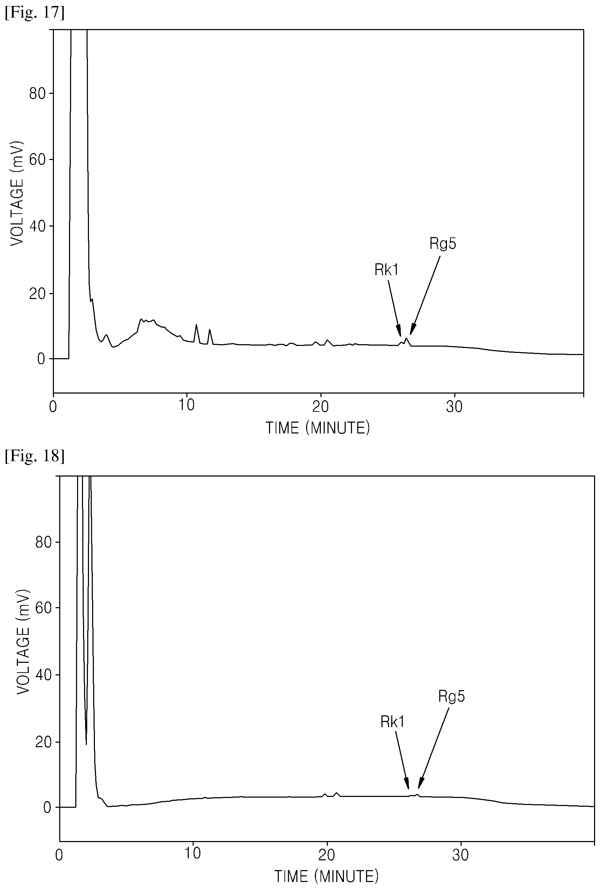

FIG. 17 is a HPLC chromatogram analyzing ginsenoside constitutions in a microwave-irradiated process product that is extracted by using an extraction solvent after irradiating microwaves to a ginseng extract at a temperature of 180°C at 100 W for 30 minutes.

FIG. 18 is a HPLC chromatogram analyzing ginsenoside constitutions in a microwave-irradiated process product that is extracted by using an extraction solvent after irradiating microwaves to a ginseng extract at a temperature of 190°C at 100 W for 30 minutes.

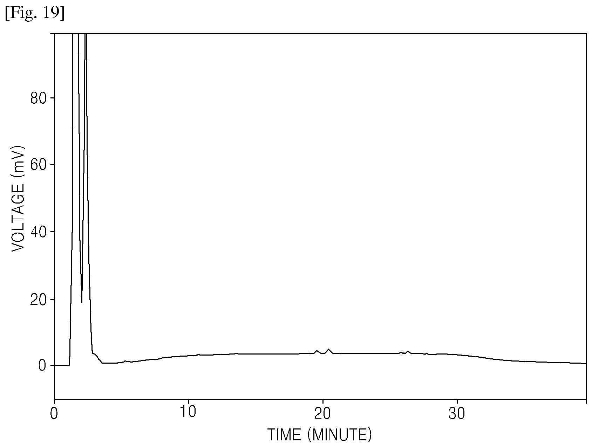

FIG. 19 is a HPLC chromatogram analyzing ginsenoside constitutions in a microwave-irradiated process product that is extracted by using an extraction solvent after irradiating microwaves to a ginseng extract at a temperature of 200°C at 100 W for 30 minutes.

Also, the results of ginsenoside contents calculated from the HPLC chromatograms are shown in Table 2.

### Table 2

**[Table 2]**

| | Re+Rg1 | Rb1 | Rc | Rb2 | Rd | 20(S)Rg3 | 20(R)Rg3 | Rk1 | Rg5 | (RG5+RK1)/Rg3 |
|---|---|---|---|---|---|---|---|---|---|---|
| Example 1 | 114.4±10.5 | 158±15.0 | 93.9±10.1 | 74.2±6.8 | 8.1±1.2 | - | - | - | - | - |
| Comparative Example 1 | 6.5±0.8 | 12.4±1.5 | 1.9±0.7 | 4.5±0.8 | 1.6±0.6 | 2.5±0.8 | 1.3±0.6 | 1.3±0.9 | 2.2±0.9 | 0.9 |
| Comparative Example 2 | 2.5±0.6 | 4.5±0.7 | 2.0±0.8 | 4.1±0.9 | 2.3±0.5 | 12.3±0.9 | 4.9±0.7 | 10.0±1.0 | 12.6±0.7 | 1.3 |
| Example 2 | 112.7±4.9 | 103.3±10.4 | 50.3±6.0 | 39.2±3.2 | 16.8±2.9 | 6.9±0.9 | 3.6±0.8 | 13.5±0.9 | 19.0±1.0 | 3.1 |
| Example 3 | 50.0±3.3 | 34.5±0.8 | 11.5±3.5 | 15.2±1.6 | 7.4±1.7 | 21.9±3.6 | 11.0±1.8 | 46.6±7.3 | 67.4±10.5 | 3.5 |
| Example 4 | 13.3±1.8 | 5.0±0.7 | 1.2±0.5 | 4.5±0.6 | 2.2±0.5 | 44.7±2.4 | 24.5±0.7 | 96.6±5.3 | 143.6±10.3 | 3.5 |
| Example 5 | - | - | - | 6.9±1.5 | - | 50.9±1.2 | 30.7±1.7 | 120.8±6.2 | 193.5±13.3 | 3.9 |
| Example 6 | - | - | - | 5.2±0.9 | - | 45.5±0.8 | 32.9±1.4 | 117.5±3.2 | 220.2±6.5 | 4.3 |
| Example 7 | - | - | - | 8.6±1.4 | - | 60.6±11.7 | 44.0±4.3 | 150.5±12.8 | 275.1±1.8 | 4.1 |
| Example 8 | - | - | - | 6.0±1.4 | - | 41.5±1.8 | 40.6±0.9 | 125.9±3.0 | 274.5±5.1 | 4.9 |
| Example 9 | - | - | - | 7.1±1.5 | - | 51.7±2.3 | 38.4±2.5 | 126.6±15.7 | 245.9±22.4 | 4.1 |
| Example 10 | - | - | - | 5.8±2.1 | - | 27.0±1.7 | 32.8±1.3 | 96.8±3.6 | 244.4±8.2 | 5.7 |
| Example 11 | 116.2±2.7 | 172.5±8.3 | 92.2±9.3 | 53.5±5.5 | 6.2±0.9 | 1.9±0.6 | 1.5±0.7 | 1.5±0.6 | 1.9±0.7 | 0.97 |
| Example 12 | 93.7±4.4 | 128.7±2.5 | 66.3±4.3 | 43.6±2.9 | 5.2±0.8 | 6.2±0.6 | 4.6±0.8 | 5.3±0.7 | 8.1±0.5 | 1.24 |
| Example 13 | - | - | - | - | - | 51.4±2.9 | 11.9±3.2 | 85.1±3.6 | 144.8±5.8 | 3.63 |
| Example 14 | - | - | - | - | - | 11.45±1.0 | 13.1±2.4 | 79.6±2.2 | 194.4±4.7 | 11.17 |
| Example 15 | - | - | - | - | - | - | - | 1.9±1.5 | 4.6±4.4 | Infinite |
| Example 16 | - | - | - | - | - | - | - | 0.9±0.5 | 2.6±1.0 | Infinite |
| Example 17 | - | - | - | - | - | - | - | - | - | - |

### Table 3

**[Table 3]**

| | A content of Rg3, Rk1, and Rg5 with respect to a weight of Rb1, Rc, Rb2, Rd, Rg3, Rk1, and Rg5 (%) | A content of Rg3, Rk1, and Rg5 with respect to a weight of Re, Rg1, Rb1, Rc, Rb2, Rd, Rg3, Rk1, and Rg5 (%) |
|---|---|---|
| Example 1 | 0 | 0 |
| Comparative Example 1 | 26.35 | 21.35 |
| Comparative Example 2 | 75.52 | 72.10 |
| Example 2 | 17.03 | 11.77 |
| Example 3 | 68.17 | 55.33 |
| Example 4 | 96.00 | 92.19 |
| Example 5 | 98.29 | 98.29 |
| Example 6 | 98.77 | 98.77 |
| Example 7 | 98.40 | 98.40 |
| Example 8 | 98.77 | 98.77 |
| Example 9 | 98.49 | 98.49 |
| Example 10 | 98.57 | 98.57 |
| Example 11 | 2.05 | 1.52 |
| Example 12 | 9.03 | 6.69 |
| Example 13 | 100 | 100 |
| Example 14 | 100 | 100 |
| Example 15 | 100 | 100 |
| Example 16 | 100 | 100 |
| Example 17 | 0 | 0 |

In Table 2, each number indicates µg per 1 mg of the extract. Contents of main ginsenosides Rg3 (20(S)+20(R)), Rg5, and Rk1 with respect to a weight of Rb1, Rc, Rb2, Rd, Rg3, Rk1, and Rg5 or a weight of Re, Rg1, Rb1, Rc, Rb2, Rd, Rg3, Rk1, and Rg5 in Table 2 are calculated, and the results are shown in Table 3.

As shown in FIGS. 1 to 3 and Tables 2 and 3, when Rb1, Rb2, Rc, Rd, Rg1, and Re as main ginsenosides that contain ginseng are heat-treated at a temperature of 120°C, a glucose, which is a glycoside located at position 20, may be dissociated, and subsequently dehydration may occur at position 20 so that Rb1, Rb2, Rc, Rd, Rg1, and Re are converted to Rg3, Rg5, and Rk1 (compare FIGS. 3 and 5). Also, the remaining ginsenoside of a white ginseng whose the glycoside is not completely dissociated was detected between 8 minutes and 14 mninutes.

As shown in Examples 2 to 10 of Table 2, when microwave is irradiated at a temperature of 150°C under a pressure of 20 atm, a content of ginsenoside Rg3, Rk1, and Rg5 increased proportional to a microwave irradiation time. In this regard, a content of Rg3, Rk1, and Rg5 with respect to a weight of Rb1, Rc, Rb2, Rd, Rg3, Rk1, and Rg5 or a weight of Re, Rg1, Rb1, Rc, Rb2, Rd, Rg3, Rk1, and Rg5 in the microwave-irradiated process product increased unexpectedly and significantly to 90% or more after 30 mninutes of reaction, and a ratio of (Rg5 + Rk1)/(Rg3) increased unexpectedly to 3.0 or higher. A content of Rg3, Rk1, and Rg5 with respect to a weight of Rb1, Rc, Rb2, Rd, Rg3, Rk1, and Rg5 or a weight of Re, Rg1, Rb1, Rc, Rb2, Rd, Rg3, Rk1, and Rg5 is an amount that is 200% or more increased than that of a simple heat-treated process white ginseng.

In addition, as shown in Examples 13 to 16 of Table 3, when microwave is irradiated at a temperature in a range of 150°C to 190°C under a pressure of 20 atm, a content of Rg3, Rk1, and Rg5 with respect to a weight of Rb1, Rc, Rb2, Rd, Rg3, Rk1, and Rg5, or a weight of Rg1, Re, Rb1, Rc, Rb2, Rd, Rg3, Rk1, and Rg5 unexpectedly and significantly increased to 95% or more, for example, 98% or more, or to 100%. A ratio of ginsenoside (Rg5 + Rk1)/(Rg3) was also 3.0 or higher.

According to the results above, when microwave is irradiated to a ginseng extract or a ginseng itself, main ginsenosides Rb1, Rb2, Rc, Rd, and Re may be efficiently converted to pharmaceutically effective ginsenosides Rg3, Rg5, and Rk1. For example, a microwave-irradiated process product containing 90% or more, 95% or more, 98% or more, or 100% of Rg3, Rk1, and Rg5 with respect to a weight of ginsenoside Rb1, Rc, Rb2, Rd, Rg3, Rk1, and Rg5, or a weight of Rg1, Re, Rb1, Rc, Rb2, Rd, Rg3, Rk1, and Rg5 was obtained. Particularly, a ratio of ginsenoside (Rg5 + Rk1)/(Rg3) is significantly higher than a simple heat-treated process product. Such effect is a significant effect that is completely unpredicted compared to a conventional art.

### 1. Preparation of pharmaceutical formulation

### 1. Preparation of powders

Active ingredient 2 g
Lactose 1 g
Powders were prepared by mixing the above ingredients and filling a sealed package with the resultant mixture.

### 2. Preparation of tablet

Active ingredient 100 mg
Corn starch 100 mg
Lactose 100 mg
Magnesium stearate 2 mg
Tablets were prepared by mixing the above ingredients and tableting the resultant mixture using a conventional method of preparing a tablet.

### 3. Preparation of capsule

Active ingredient 100 mg
Corn starch 100 mg
Lactose 100 mg
Magnesium stearate 2 mg
Capsules were prepared by mixing the above ingredients and filling a gelatin capsule with the resultant mixture using a conventional method of preparing capsules.

### 4. Preparation of pill

Active ingredient 1 g
Lactose 1.5 g
Glycerin 1 g
Xylitol 0.5 g
Pills were prepared by mixing the above ingredient so that the pills contained 4 g of the ingredients per pill by using a conventional method.

### 5. Preparation of granule

Active ingredient 150 mg
Soybean extract 50 mg
Glucose 200 mg
Starch 600 mg
The above ingredients were mixed together, 100 mg of 30% ethanol was added to the mixed ingredients, the resultant mixture was dried at 60°C to form granules, and a packet was filled with the granules.

### 2. Preparation of health food

### 1. Preparation of tomato ketchup and sauce

0.2 to 1.0 parts by weight of the active ingredient was added to tomato ketchup and sauce to prepare tomato ketchup or sauce for health improvement.

### 2. Preparation of flour food

0.5 to 5.0 parts by weight of the active ingredient was added to flour, and bread, cake, cookies, cracker, and noodles were prepared using the resultant mixture, thereby completing the preparation of foods for health improvement.

### 3. Preparation of soup and gravies

0.1 to 5.0 parts by weight of the active ingredient was added to soup and gravy to prepare soup and gravy of processed meat products and noodles for health improvement.

### 4. Preparation of ground beef

10 parts by weight of the active ingredient was added to ground beef to prepare ground beef for health improvement.

### 5. Preparation of dairy products

5 to 10 parts by weight of the active ingredient was added to milk and a variety of dairy products such as butter and ice cream were prepared using the resultant milk.

### 6. Preparation of natural food products

Brown rice, barley, glutinous rice, and adlay were pregelatinized using a known method and dried, and the dried resultant mixture was roasted and pulverized using a pulverizer to prepare powders having a particle diameter of 60 mesh. Separately, black beans, black sesame seeds, and perilla seeds were steamed using a known method and dried, and the dried resultant mixture was roasted and pulverized using a pulverizer to prepare powder having a particle diameter of 60 mesh. The active ingredient was concentrated in a vacuum concentrator and dried using a spray convection dryer to obtain a dried material. Thereafter, the dried material was pulverized using a pulverizer to obtain a dried powder having a particle diameter of 60 mesh.

The prepared grains, seeds and nuts, and the dried powder of the extracts of Examples 1 and 2 were mixed at a mixing ratio as described below to prepare a final product:
Grains (30 parts by weight of brown rice, 15 parts by weight of adlay, and 20 parts by weight of barley),
Seeds and nuts (7 parts by weight of perilla seeds, 8 parts by weight of black beans, and 7 parts by weight of black sesame seeds),
3 parts by weight of the dried powder of a compound was separated from the extracts of Examples 1 and 2,
0.5 parts by weight of Lingzhi mushroom
0.5 parts by weight of geogen

### 3. Preparation of health beverage

### 1. Preparation of healthy drink

Active ingredient 1000 mg
Citric acid 1000 mg
Oligosaccharide 100 g
Plum concentrated extract 2 g
Taurine 1 g
A total volume with addition of purified water 900 ml

The composition as described herein was prepared by mixing ingredients relatively appropriate for beverages at a mixing ratio according to a preferred example, but the mixing ratio of the ingredients may be modified according to geographic and ethnic preferences, such as target customers, target country, usage, and the like.

### 2. Preparation of vegetable juice

5 g of the active ingredient was added to 1,000 ml of tomato or carrot juice to prepare vegetable juice for health improvement.

### 3. Preparation of fruit juice

1 g of the active ingredient was added to 1,000 ml of apple or grape juice to prepare fruit juice for health improvement.

As described above, according to the one or more embodiments of the present invention, methods of preparing a Panax spp. plant root extract are provided and the extract has a significantly high content ratio of ginsenoside Rg5+Rk1+Rg3 compared to a processed Panax spp. plant extract prepared by simple heat-treating. Thus, the processed Panax spp. plant extract may be used to manufacture a pharmaceutical composition and a health functional food composition with enhanced ginsenoside Rg5+Rk1+Rg3 effect, particularly Rg5 and Rk1 effect.

### Industrial Applicability

As described above, according to the one or more embodiments of the present invention, methods of preparing a processed Panax spp. plant root extract are provided and the extract has a significantly high content ratio of ginsenoside Rg5+Rk1+Rg3 compared to a processed Panax spp. plant extract prepared by simple heat-treating. Thus, the extracts prepared by the methods may be used to manufacture a pharmaceutical composition and a health functional food composition with enhanced ginsenoside Rg5+Rk1+Rg3 effect, particularly Rg5 and Rk1 effect.

## Claims

1. A method of preparing a Panax spp. plant root extract containing 90% or more of Rg3, Rk1, and Rg5 with respect to a weight of ginsenoside Rb1, Rc, Rb2, Rd, Rg3, Rk1, and Rg5, wherein the method comprises irradiating microwaves to a Panax spp. plant root or an extract thereof, wherein the irradiating of the microwaves is performed under pressure at a temperature in a range of 150°C to 190°C for 30 minutes to 90 minutes, wherein the irradiating of the microwaves is performed under a pressure in a range of 2 atm to 100 atm and the Panax spp. plant extract has a ratio of (Rg5 + Rk1)/(Rg3) that is at least 2.

2. The method of claim 1, wherein the Panax spp. plant extract has a ratio of (Rg5 + Rk1)/(Rg3) that is at least 3.

3. The method of claim 1, wherein the Panax spp. plant is Panax ginseng, Panax quinquefolia, Panax notoginseng, Panax japonica, Panax trifolia, Panax pseudoginseng, Panax vietnamensis, a cultured root thereof, a heat-treated or enzyme-treated process product thereof, or a combination thereof.

4. The method of claim 1, wherein the extract of Panax species plant root comprises a crude extract of water, C1-C4 alcohol, or a mixture thereof of Panax species plant; a solvent fraction of n-hexane, methylenechloride, ethylacetate, butanol, or a mixture thereof of the crude extract; or a purified material of the solvent fraction.

## Patentansprüche

1. Verfahren zur Zubereitung eines Panax spp. Pflanzenwurzelextrakts, der 90% oder mehr Rg3, Rk1 und Rg5 in Bezug auf ein Gewicht von Ginsenosid Rb1, Rc, Rb2, Rd, Rg3, Rk1 und Rg5 aufweist, wobei das Verfahren das Bestrahlen einer Panax spp. Pflanzenwurzel oder eines Extrakts davon mit Mikrowellen umfasst, wobei das Bestrahlen mit Mikrowellen unter Druck bei einer Temperatur im Bereich von 150 °C bis 190 °C über 30 Minuten bis 90 Minuten ausgeführt wird, wobei das Bestrahlen mit Mikrowellen bei einem Druck in einem Bereich von 2 atm bis 100 atm ausgeführt wird, und wobei der Panax spp. Pflanzenextrakt ein Verhältnis von (Rg5 + Rk1) / (Rg3) von mindestens 2 aufweist.

2. Verfahren nach Anspruch 1, wobei der Panax spp. Pflanzenextrakt ein Verhältnis von (Rg5 + Rk1) / (Rg3) von mindestens 3 aufweist.

3. Verfahren nach Anspruch 1, wobei die Panax spp. Pflanze Panax gingseng, Panax quinquefolius, Panax notogingseng, Panax japonicus, Panax trifolius, Panax pseudogingseng, Panax vietnamensis, eine kultivierte Wurzel davon, ein wärmebehandeltes oder enzymbehandeltes Verfahrensprodukt davon oder eine Kombination davon ist.

4. Verfahren nach Anspruch 1, wobei der Extrakt der Panax Pflanzenwurzel einen rohen Extrakt von Wasser, C1-C4-Alkohol oder ein Gemisch davon der Panax Pflanze umfasst; einen Lösungsmittelanteil von n-Hexan, Methylenchlorid, Ethylacetat, Butanol oder ein Gemisch davon des rohen Extrakts; oder ein aufgereinigtes Material eines Lösungsmittelanteils.

## Revendications

1. Procédé de préparation d'un extrait de racine végétale de Panax spp. contenant 90 % ou plus de Rg3, Rk1 et Rg5 par rapport à un poids de ginsénoside Rb1, Rc, Rb2, Rd, Rg3, Rk1 et Rg5, le procédé comprenant l'étape consistant à irradier des micro-ondes sur la racine de Panax spp. ou un extrait de celle-ci, l'irradiation des micro-ondes étant effectuée sous pression à une température comprise entre 150 °C et 190 °C pendant 30 à 90 minutes, l'irradiation des micro-ondes étant effectuée sous une pression comprise entre 2 atm et 100 atm et l'extrait de Panax spp. ayant un rapport de (Rg5 + Rk1)/(Rg3) qui est au moins égal à 2.

2. Procédé selon la revendication 1, l'extrait végétal de Panax spp. ayant un rapport de (Rg5 + Rk1)/(Rg3) qui est d'au moins 3.

3. Procédé selon la revendication 1, la plante Panax spp. étant du Panax ginseng, Panax quinquefolia, Panax notoginseng, Panax japonica, Panax trifolia, Panax pseudoginseng, Panax vietnamensis, une racine cultivée de ceux-ci, un produit de procédé traité à la chaleur ou traité par enzyme de ceux-ci, ou une combinaison de ceux-ci.

4. Procédé selon la revendication 1, l'extrait de racine végétale de l'espèce Panax comprenant un extrait brut d'eau, de l'alcool en C1-C4 ou un mélange de ceux-ci de plante de l'espèce Panax ; une fraction solvant de n-hexane, dichlorométhane, acétate d'éthyle, butanol, ou d'un mélange de ceux-ci de l'extrait brut ; ou un matériau purifié de la fraction solvant.
